# EUROPEAN PATENT SPECIFICATION

(11) **EP 0 474 188 B1**
(45) Date of publication and mention of the grant of the patent: **18.06.1997**
(21) Application number: 91114842.7
(22) Date of filing: 03.09.1991
(51) Int. Cl.: C07C 5/52, C07C 41/09, C07C 41/06

(54) **Methyl-tertiaryalkyl ether production**
Methyltertiäralkyletherherstellung
Production de méthyle tertiaire alkyle éther

(30) Priority: 04.09.1990 US 577182
(43) Date of publication of application: 11.03.1992
(73) Proprietor: PHILLIPS PETROLEUM COMPANY, Bartlesville Oklahoma 74004 (US)
(72) Inventor: Hensley, Harvey Dean, Bartlesville, OK 74006 (US); Anderson, Richard Lee, Bartlesville, OK 74006 (US); Olbrich, Michael Eugene, Naperville, IL 60563 (US)
(74) Representative: Geissler, Bernhard, Dr.jur., Dipl.-Phys.

(56) References cited:
- EP-A- 0 103 870
- EP-A- 0 221 332
- AU-B- 563 178
- GB-A- 2 010 323
- GB-A- 2 050 379
- US-A- 4 152 365
- DATABASE WPI Week 8942, Derwent Publications Ltd., London, GB; AN 89-306547 & JP-A-1 228 922 (PHILLIPS PETROLEUM CO)
- DATABASE WPI Week 8942, Derwent Publications Ltd., London, GB; AN 89-306547 & JP-A-1 228 922

## Description

This invention relates to a process for the production of tertiary-alkyl ether compounds by reacting an olefin and an organic hydroxy containing compound. In another aspect it relates to an integrated process which advantageously joins isoolefin production and ether production.

### BACKGROUND OF THE INVENTION

It is well known that tertiary-alkyl ether compounds can be prepared by reacting a primary alcohol with an isoolefin having a double bond on a tertiary carbon atom, such as the catalytic reaction of methanol with isobutene and isopentenes to form methyl tertiary-butyl ether (MTBE) when reacting isobutene, and methyl tertiary-amyl ether (MTAE) when reacting isopentene. When ethanol is used in lieu of methanol, ethyl tertiary butyl ether (ETBE) and ethyl tertiary-amyl ether (ETAE) respectively, are formed.

Interest in the production of tertiary-alkyl ethers, which can be used for high octane blending components for gasoline, comes primarily from increased demand for higher octane gasoline with lower Reid vapor pressure. This demand has been stimulated by government regulations concerning the environment which restrict the use of lead as an octane improver for gasoline.

EP-A1-0 221 332 relates to a transhydrogenation process for the conversion of alkanes to alkenes with high yields and extended catalyst life between regenerations.

It discloses the use of a catalyst consisting of an alumina support of a specific particle size and surface area which is impregnated with chromic acid and thus has a loading of chromium. This catalyst is used for the transhydrogenation of a blend of isobutane and ethylene to result in isobutene and ethane, as well as a transhydrogenation of a blend of propane and ethylene resulting in propene and ethane.

AU-B-563 178 relates to a process for the manufacture of methyl t-butyl ether (MTBE) and, in particular, to an integrated process for the manufacture of MTBE involving the use of a transhydrogenation reaction to produce isobutene. A mixture of isobutane and ethylene is used in the transhydrogenation reaction. The transhydrogenation catalyst employed is chromium oxide on alumina.

It is, therefore, an object of this invention to convert low octane, high Reid vapor pressure hydrocarbons to high octane, low Reid vapor pressure organic compounds.

It is another object of this invention to provide a process for simultaneously dehydrogenating and hydrogenating a hydrocarbon feed stream containing an isoparaffin having four or five carbon atoms per molecule and a n-olefin having an equal number of carbon atoms, to a product stream containing an isoolefin and an n-paraffin.

It is a further object of this invention to provide an improved process for the commercial production of high octane blending components for gasoline.

It is a still further object of this invention to provide commercial processes which allow refiners to increase production of tertiary ethers by converting C₄ or C₅ hydrocarbons to their respective ethers.

### SUMMARY OF THE INVENTION

In accordance with one aspect of this invention, there is provided a process for simultaneous hydrogenation of an olefin and dehydrogenation of a paraffin in a combination hydrogenation/dehydrogenation unit having a single reaction zone.

In a preferred embodiment, the simultaneous hydrogenation/ dehydrogenation process is utilized in an integrated process which joins etherification with a companion process for forming isoolefins. In the integrated process, the isoolefin is formed in the combination hydrogenation/dehydrogenation reactor from a feed stream comprising a structural mixture of hydrocarbons. As used herein, a structural mixture of hydrocarbons comprises a mixture containing at least an n-olefin and an isoparaffin and may contain significant amounts of n-paraffin and isoolefin with a substantial portion of the constituents having four or five carbon atoms per molecule. The processes of hydrogenation/dehydrogenation and etherification are joined by reacting the isoolefin produced in the combination hydrogenation/dehydrogenation reactor with methanol or ethanol to produce a tertiary-alkyl ether compound. The integrated process comprises the steps of:
(a) passing a structurally mixed hydrocarbon feed stream containing an isoparaffin having four or five carbon atoms per molecule and an n-olefin, having a number of carbon atoms per molecule equal to said isoparaffin, to a combination hydrogenation/dehydrogenation unit;
(b) contacting said mixed hydrocarbon feed stream with a supported Group VIII noble metal catalyst in said combination unit under conditions sufficient for simultaneous conversion of a portion of said isoparaffin to an isoolefin and a portion of said n-olefin to an n-paraffin;
(c) withdrawing reaction product in a stream from said combination unit and passing at least a portion of said reaction product stream to an ether forming unit;
(d) providing a primary alcohol containing one or two carbon atoms per molecule to said ether forming unit via an alcohol feed stream; and
(e) reacting said isoolefin and said primary alcohol in said ether forming unit to form tertiary alkyl ether, and withdrawing tertiary alkyl ether from said ether forming unit in an ether product stream,
   in particular wherein said isoparaffin is isobutane and said n-olefin is n-butene and said ether unit is an MTBE unit and said tertiary alkyl ether is MTBE.

Further aspects and additional advantages of the present invention will be apparent from the following detailed description of the preferred embodiments of the invention as illustrated by the drawings in which:

### BRIEF DESCRIPTION OF THE DRAWINGS

FIG.'s 1 and 2 illustrate simplified process flow schemes using a combination hydrogenation/dehydrogenation process step for C₄ hydrocarbons in the production of MTBE or ETBE according to this invention.

FIG's. 3-6 illustrate simplified process flow schemes using a combination hydrogenation/dehydrogenation process step for C₄ hydrocarbons in the production of MTBE or ETBE and alkylate according to this invention.

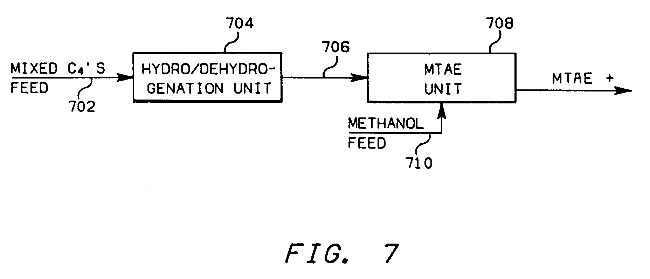

FIG. 7, illustrates a simplified process flow scheme using a combination hydrogenation/dehydrogenation process step for C₅ hydrocarbons in the production of MTAE or ETAE according to this invention.

### DETAILED DESCRIPTION OF THE PREFERRED EMBODIMENTS

In preferred embodiments illustrated in the drawing FIG.'s 1 and 2, this invention involves combinations of several processes which are employed in a unique configuration to achieve the objects of this invention. Such processes operate on a feed stream comprising a structural mixture of C₄ hydrocarbons, and may include distillation, absorption, and isomerization as illustrated in the specific embodiments in FIG.'s 1 and 2. In other preferred embodiments illustrated in FIG's. 3-6, a variety of process configurations are disclosed in which an alkylation process step is added to the general processes disclosed in FIG's. 1 and 2, so that a portion of the mixed hydrocarbon feed stream may be converted to alkylate. In still other embodiments illustrated in FIG. 7, the feed stream comprises a mixture of C₅ hydrocarbons which is converted to higher octane components.

An essential feature of all the disclosed embodiments of the present invention is directed to integrating a combination hydrogenation/dehydrogenation step, which produces an isoolefin from an isoparaffin, into an etherification process. In the preferred embodiments the hydrocarbon feed meterial is a non-aromatic hydrocarbon having at least four carbon atoms.

The combination hydrogenation/dehydrogenation reactions are carried out in a unit having a single reaction zone, in the presence of a single dehydrogenation or reforming catalyst, such as platinum and tin on a zinc aluminate support. The catalyst composition, which is employed in the hydrogenation/dehydrogenation step of this invention, can be prepared by any suitable method, such as is well known by those familiar in the art. The preparation comprises combining, in any suitable manner, (i) a Group IIA metal aluminate spinel (i.e. aluminate spinel of Be and/or Mg and/or Ca and/or Sr and/or Ba), or a Group IIB metal aluminate spinel (i.e. aluminate spinel of Cd and/or Zn), or mixture of two or more of the above metal aluminate spinels; (ii) Group VIII metal and/or compounds thereof, and (iii) compounds of Ge and/or Sn and/or Pb.

Aluminate spinels, as referred to herein, are compounds of the formula M(AlO₂)₂ or MO · Al₂O₃ where M is a metal of Group IIA or IIB of the Periodic Table (as defined in Webster's New Collegiate Dictionary, 1977, page 852) with a valence of 2, such as Zn, Mg, Be, Ca and the like. The preparation of these aluminate spinels is described in numerous patents, such as U.S. Patent No. 3,641,182; 3,670,044; 3,880,776; 3,894,110; and 4,152,365. In a preferred embodiment tin oxide is incorporated into the aluminate spinel. In another preferred embodiment, component (i) comprises zinc aluminate as a major component and calcium aluminate as a binder (generally present at 5-25 wt. %).

In the presently preferred method of catalyst preparation, the metal aluminate spinel is prepared by ball-milling appropriate amounts of zinc oxide and alumina and, optionally, tin oxide (SnO and/or SnO₂), and calcining (preferably by heating in air) the mixture at a sufficiently higher temperature for a sufficient length of time to form the spinel. Preferably, the spinel component is used as support material, which is impregnated with component (ii) and with component (iii) in any suitable manner, either sequentially in any order, or simultaneously, as has been described in the above-cited patents.

The components of the catalyst composition generally are present at the following levels: 80-98 weight-% of Group IIA and/or IIB metal aluminate spinel (preferably zinc aluminate); 0.05-5 weight-% of Group VIII metal (preferably Pt); and 0.1-5 weight-%. Group IVA metal (preferably Sn which is present as an oxide). It is understood that additional components which are beneficial for catalyzing the hydrogenation/dehydrogenation operation may be present in small amounts, such as Re, Au, Ag, alkali metals, Ce, and the like. Suitable inorganic binder materials (such as amorphous alumina) may also be present. Generally, the surface area of the composition of matter (after calcination) is in the range of from 5 to 100 m²/g (determined by nitrogen adsorption in accordance with the BET method).

In this combination hydrogenation/dehydrogenation unit the heat evolved in the exothermicity of the butene hydrogenation reaction is balanced against the nearly equal heat absorbed in the endothermicity of the isobutane dehydrogenation reaction. The reaction in the hydrogenation/dehydrogenation unit may be represented by the following equation for a preferred embodiment:

n-butene + isobutane → butane + isobutene

Of course, the reaction conditions of temperature and pressure must be such as to permit both hydrogenation and dehydrogenation reactions to proceed. It has been found that the heat requirements of the combined reaction can be essentially satisfied through internal generation with the reaction temperature generally in the range of from 316°C (600°F) to 593°C (1100°F). The preferred pressure is generally in the range of (2.7 - 6.2) x 10⁵ Pa (25-75 psig) but can be substantially higher.

In the preferred combination hydrogenation/dehydrogenation process step, a vaporized C₄ hydrocarbon feed stream, optionally mixed with steam, is preheated and passed through a reactor containing a fixed bed of the catalyst composition (which can be in any suitable form such as granules, pellets, spheres and the like). The liquid hourly space velocity of the vaporized structurally mixed hydrocarbon feed (excluding steam) generally is in the range of from 0.5 - 4.0. In another embodiment, a C₅ hydrocarbon feed stream is converted.

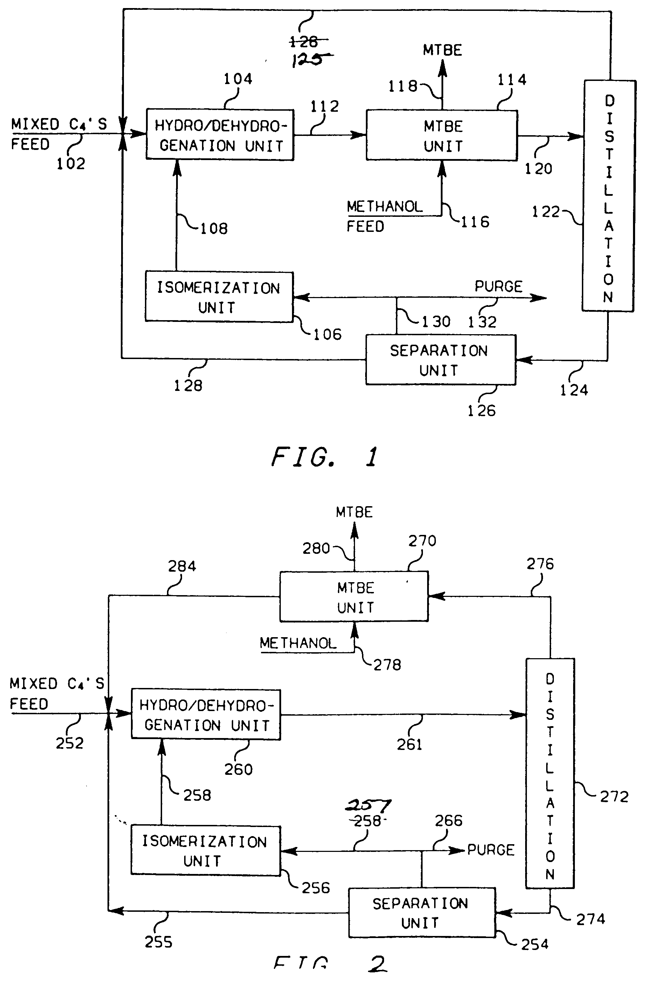

Referring specifically now to FIG. 1, there is illustrated a preferred embodiment of this invention in which a structurally mixed C₄ hydrocarbon feed stream may be converted to a tertiary alkyl ether such as MTBE (methyl tertiary-butyl ether). Depending on the concentration of isobutane in the feed stream, it may be necessary, however, to add additional isobutane from an external source to achieve full conversion of the feed stream to MTBE.

A structurally mixed C₄ hydrocarbon feed stream comprising n-butene, n-butane, isobutene, and isobutane from a catalytic cracking plant or other source, is provided via conduit 102 to a combination hydrogenation/dehydrogenation unit 104 along with an isobutane stream via conduit 108 supplied from an isomerization unit 106.

The reaction product from the combination hydrogenation/dehydrogenation unit 104 comprising primarily isobutene but also containing significant amounts of butene-1, butene-2, n-butane and isobutane is charged to a conventional ether forming unit such as MTBE unit 114 via conduit 112, along with methanol feed which is supplied via conduit 116. From unit 114, which includes a reactor and a distillation column, MTBE is recovered via conduit 118 for use in gasoline blends as on octane enhancer. The MTBE operation reacts isobutene with methanol to make MTBE. The ether forming reaction of the MTBE etherification process step of this invention is known in the art both generally and in many of its details. Reference is made to U.S. Patent No. 3,846,088 in which such an ether production, particularly for the production of MTBE, is described. Also, the above-described ether forming reaction can be modified by persons having ordinary skill in the art so as to produce ethyl tertiary-butyl ether (ETBE) by employing ethanol in lieu of methanol.

A residual stream of remaining unreacted constituents, enriched in linear butenes, is withdrawn from the MTBE unit 114 via conduit 120 and is passed to a distillation tower 122. In tower 122 n-butane and butene-2 are separated from the feed mixture and withdrawn as a bottoms stream via conduit 124. An overhead distillation product comprising isobutane, isobutene and butene-1 is withdrawn from distillation tower 122 and recycled to the combination reactor 104 via conduit 125.

The distillation bottoms stream flowing in conduit 124 is passed to a separations unit 126, which may provide either an adsorption or extractive distillation step to separate n-butane and butene-2. The butene-2 fraction is withdrawn from separation unit 126 and recycled directly to the combination reactor 104 via conduit 128. n-Butane is withdrawn from separation unit 126 via conduit 130 and passed to an isomerization unit 106, where isobutane is produced therefrom. The isomerization unit 106 is a conventional catalytic unit for the conversion of n-butane to isobutane. A purge stream withdrawn from separation unit 126 via conduits 130 and 132 is needed to remove the saturates which are present in the mixed C₄ feed introduced via conduit 102.

The mixed C₄ feed stream in conduit 102 may be introduced at various other points in the process network, depending upon its composition. The feed stream in the illustrated process should be introduced into a stream having a similar composition. For example, if the mixed C₄ feed stream contains no iso-compounds, it should be introduced in conduit 124. If the mixed C₄ feed stream contains substantial isobutene, it may be introduced into the MTBE unit 114.

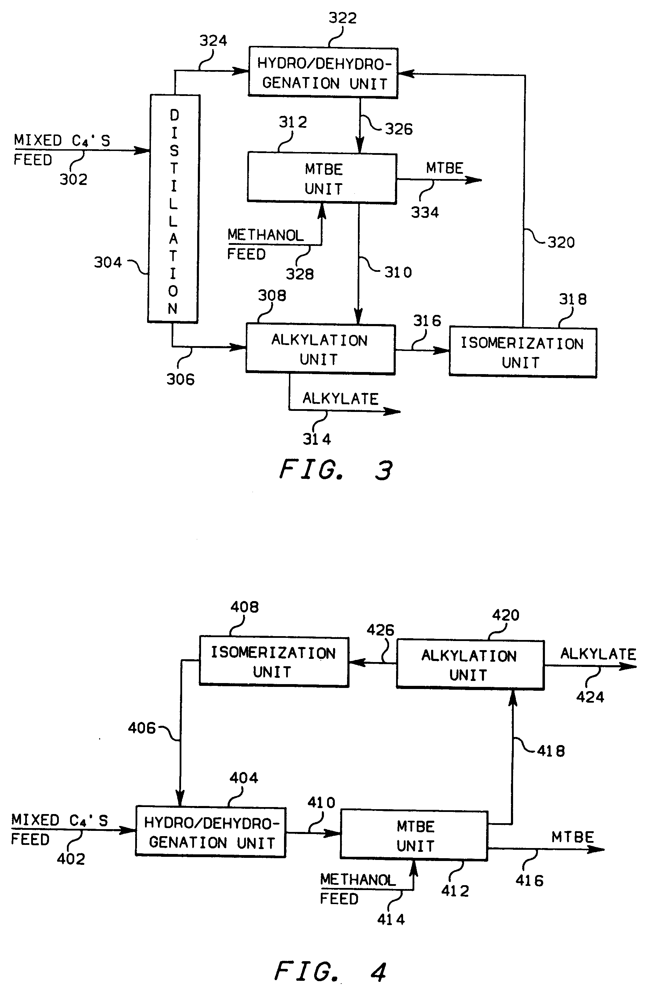

The embodiment of the present invention illustrated in Fig. 2 employs units which operate in the same manner as the corresponding units in FIG. 1, and the process flow illustrated in FIG. 2 differs only slightly from that of FIG. 1. Referring now to FIG. 2, a structurally mixed C₄ hydrocarbon feed stream comprising n-butene, n-butane, isobutene, and isobutane from a catalytic cracking plant or other source, is provided via conduit 252 to a combination hydrogenation/dehydrogenation unit 260, along with an isobutane stream via conduit 258 supplied from an isomerization unit 256. The effluent from the hydrogenation/dehydrogenation unit 260 feeds the distillation column 272 via conduit 261 instead of feeding to the MTBE unit as illustrated in FIG. 1. The overhead from the distillation unit 272 in FIG. 2, which is enriched in isobutene and also contains isobutane and butene-1, is then fed to the MTBE unit 270 via conduit 276 along with methanol feed, which is supplied via conduit 278. In the MTBE unit 270, MTBE is produced and separated via conduit 280, and the unreacted C₄'s are recycled to hydrogenation/dehydrogenation unit 260 via conduit 284. The remainder of the process flow including flow through separation unit 254 via conduit 274, then through isomerization unit 256 via conduit 257 and recycle streams via conduits 284 and 255 is identical to the process flow illustrated in FIG. 1. As stated above in reference to FIG. 1, the mixed C₄ feed stream in FIG. 2 is preferably introduced into a stream with a composition most resembling the composition of the mixed C₄ feed stream.

The processes illustrated in FIG's. 1-2 generally require a purge of saturated compounds essentially equivalent to the amount of saturated compounds present in the mixed C₄ feed stream. A purge stream withdrawn from separation unit 254 via conduit 266 is needed to remove the saturates which are present in the mixed C₄ feed introduced via conduit 252. Total utilization of the mixed C₄ feed stream for octane improvement can be accomplished by the addition of an alkylation unit to the process schemes illustrated in FIG's. 1 and 2. Example configurations are shown in FIG's. 3-6.

The alkylation unit illustrated in FIG's. 3-6 is well known. U.S. Patent Nos. 3,213,157, 3,211,536, and 3,309,882 describe such alkylation processes which employ liquid hydrofluoric (HF) acid as the catalyst. These patents are incorporated herein by reference. In the conventional HF alkylation reaction liquid isoparaffin and liquid olefin are contacted with liquid HF catalyst to form a reaction mixture. After liquid-liquid phase separation of this reaction mixture an alkylate is removed from the organic phase as one product of the process. The olefins useful in HF alkylation reactions are those having three to five carbon atoms. The paraffins used for alkylation reaction are generally isoparaffins having four to six carbon atoms, isobutane being particularly preferred.

Referring now to FIG. 3, a feed stream identical to the feed stream described in reference to FIG. 1 is provided to distillation tower 304 via conduit 302. In distillation tower 304 n-butane and butene-2 are separated from the feed mixture and withdrawn in a bottoms stream and passed via conduit 306 to an alkylation unit 308. Also introduced into the alkylation unit 308 via conduit 310 is an isobutane, butene-1 and butene-2 containing stream from an ether forming unit such as MTBE unit 312. From the alkylation unit an alkylate product stream is withdrawn via conduit 314 and a paraffin (n-butane) stream is withdrawn via conduit 316. The n-paraffin stream in conduit 316 is passed to an isomerization unit 318 where the n-butane is converted to isobutane. The isomerization reaction product stream containing isobutane flowing in conduit 320 is passed to the combination hydrogenation/dehydrogenation 322 via conduit 320. Also introduced into the hydrogenation/dehydrogenation unit 322, is an overhead distillation product comprising isobutane, isobutene and butene-1 which is withdrawn from distillation tower 304 via conduit 324. The product stream, enriched in isobutene, is withdrawn from the combination hydrogenation/dehydrogenation unit 322 and passed to the MTBE unit 312 via conduit 326, where isobutene is reacted with methanol supplied via conduit 328 to form MTBE. If ethanol is changed in lieu of methanol, ETBE is formed in unit 312. From unit 312, MTBE is withdrawn via conduit 334, and a residual stream of remaining unreacted constituents is withdrawn via conduit 310 and passed to alkylation unit 308.

Referring now to FIG. 4, a feed stream, identical to the feed stream described in reference to FIG. 1 is provided to a combination hydrogenation/dehydrogenation unit 404 via conduit 402. Also charged to combination unit 404 via conduit 406 is an isobutane containing stream supplied from the isomerization unit 408. Reaction products comprising isobutene and n-butane along with unreacted n-butene and isobutane are withdrawn from combination unit 404 via conduit 410 and passed to an ether forming unit such as MTBE unit 412. In MTBE unit 412 isobutene is reacted with methanol supplied via conduit 414 to produce MTBE. From unit 412, MTBE product is withdrawn via conduit 416, and a residual stream of remaining unreacted constituents comprising essentially n-butane, and n-butenes and isobutane are withdrawn via conduit 418 and passed to the alkylation unit 420. From alkylation unit 420 an alkylate product is withdrawn via conduit 424, and n-butane is withdrawn via conduit 426 and passed to isomerization unit 408.

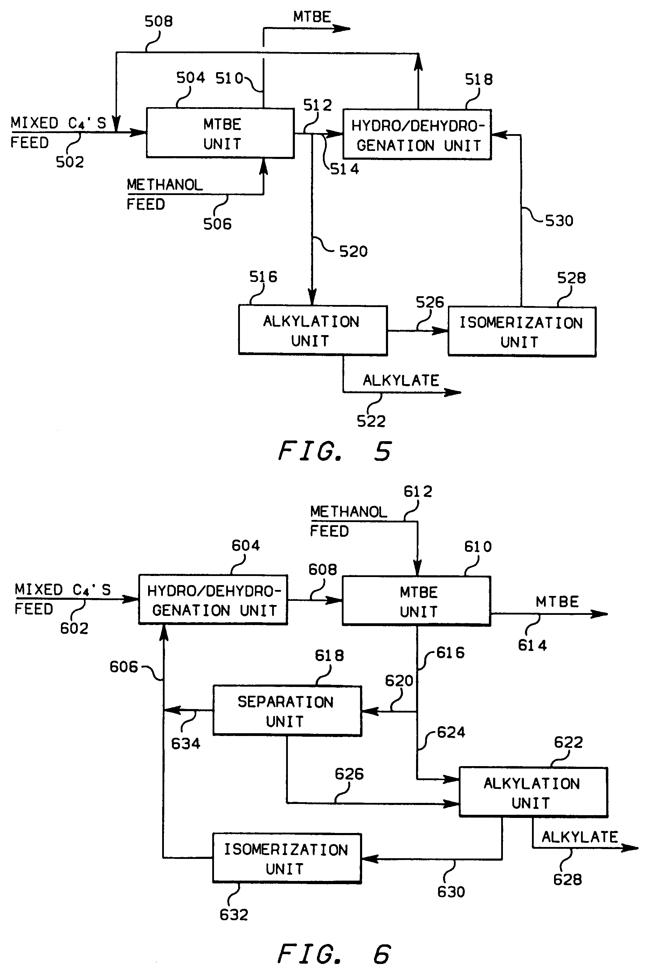

Referring now to FIG. 5, a feed stream identical to the feed stream described in reference to FIG. 1 is provided to an ether forming reactor such as MTBE unit 504 via conduit 502. Also charged to the MTBE unit 504 via conduit 506 is a methanol feed stream, and a recycle stream via conduit 503 enriched in isobutene. The isobutene and methanol are reacted in unit 504 to produce MTBE which is withdrawn via conduit 510. Also withdrawn from unit 504 is a stream containing n-butene, and isobutane via conduit 512. The stream flowing in conduit 512 is divided with a first portion passed to a hydrogenation/dehydrogenation unit 518 via conduit 514, and a second portion passed to an alkylation unit 516 via conduit 520. Alkylate is withdrawn from alkylation unit 516 via conduit 522, and n-butane is withdrawn via conduit 526. The n-butane flowing in conduit 526 is passed to an isomerization unit 528 where the n-butane is converted to isobutane and then passed to the combination unit 518 via conduit 530.

Referring now to FIG. 6, a feed stream identical to the feed stream described in reference to FIG. 1 is provided to a combination hydrogenation/dehydrogenation unit 604 via conduit 602. Also charged to the combination unit 604 via conduit 606 is a recycle stream containing isobutane and n-butene. The product stream containing isobutene, isobutane, n-butane and n-butene is withdrawn from the combination unit 604 and passed to an ether forming unit such as MTBE unit 610 via conduit 608. A methanol feed stream is also provided to MTBE unit 610 via conduit 612. From MTBE unit 610 MTBE product is withdrawn via conduit 614, and a residual stream of remaining unreacted constituents containing n-butene, n-butane, and isobutane is withdrawn via conduit 616. The stream flowing in conduit 616 is divided so that a first portion is passed to a separation unit 618 via conduit 620, and a second portion is passed to alkylation unit 622 via conduit 624. Olefins are separated from paraffins in separation unit 618 and a paraffins stream comprising n-butane and isobutane is withdrawn from separation unit 618 and passed to alkylation unit 622 via conduit 626.

Alkylate product is withdrawn from alkylation unit 622 via conduit 628. Also withdrawn from alkylation unit 622 via conduit 630 is a stream comprising n-butane which is passed to isomerization unit 632. The n-butane is isomerized in isomerization unit 632 and withdrawn therefrom and recycled to the combined unit 604 via conduit 606. Now referring again to separation unit 618, the olefin stream comprising n-butene's and isobutene is withdrawn from separation unit 618 and recycled to the combination hydrogenation/dehydrogenation unit 604 via the combination of conduits 634 and 606.

Referring now to FIG. 7, a structurally mixed C₅ feed stream primarily comprised of n-pentene and also containing isopentane and n-pentane is provided to a combination hydrogenation/dehydrogenation unit 704 via conduit 702. The reaction product from the combination unit 704 comprising primarily isopentene and also containing isopentane, n-pentane and n-pentene is passed to an ether forming unit such as MTAE unit 708 via conduit 706. In MTAE unit 708 isopentene is reacted with methanol, which is provided to reactor 708 via conduit 710, to form MTAE product. If ethanol is provided in lieu of methanol ethyl tertiary-amyl ether (ETAE) is formed in unit 708. The MTAE or ETAE product is withdrawn from reactor 708 along with unreacted isopentene and methanol, and n-pentene, isopentane and n-pentane.

Other possible process configurations for the conversion of a mixed C₅ stream to MTAE or ETAE can be illustrated by replacing the MTBE units in FIG. 1 and FIG. 2 with either MTAE or ETAE units. In these processes the mixed C₅ feed can be introduced where desired. The normal, saturated C₅ compounds in the mixed C₅ feed are purged from the loop via conduit 132 in FIG. 1, or via conduit 266 in FIG. 2.

The following examples illustrate the combination hydrogenation/dehydrogenation step, which is considered to be the critical step in each of the disclosed embodiments. These examples are presented in further illustration of this invention and are not to be considered as unduly limiting the scope of this invention.

### Example 1

A blend of technical grade isobutane with research grade butene-1 was introduced into a pilot plant reactor having a length of about 0.6 m (2 ft.) and a diameter of about 5 cm (2 inches). The reactor was filled with a layer (about 35.5 cm (14 inches) high) containing about 974 grams (780 cc) of a dehydrogenation catalyst comprising platinum and tin on a zinc aluminate/calcium aluminate base. The catalyst was prepared substantially in accordance with the method described in Example I of U.S. Patent No. 4,152,365, and contained about 0.6 weight-% platinum, 1.0 weight-% tin 98.4 weight-% zinc aluminate/calcium aluminate. The feed blend comprised the following composition:

| | |
|---|---|
| isobutane | 47.79-% by weight, |
| n-butane | 1.56-% by weight, |
| n-butene-1 | 49.99-% by weight, |
| trans-2-butene | 0.10-% by weight, |
| cis-2-butene | 0.02-% by weight, |
| propane | 0.19-% by weight, |
| pentane | 0.02-% by weight, and |
| air | 0.34-% by weight |

A hydrocarbon feed stream composed of the above feed blend was contacted with the above-described catalyst in the pilot plant reactor. Generally the feed stream was passed through the reactor, both with and without steam, for a period of time required to essentially achieve equilibrium conditions. Then the hydrocarbon feed to the reactor was discontinued, the reactor was purged with steam for 5 minutes, and air was introduced into the reactor for 25 minutes at a rate of about 283 dm³/h (10 SCFH) and then for 25 minutes at a rate of about 566 dm³/h (20 SCFH) with steam flow at about 2125-g/hr, so as to regenerate the hot catalyst by burning off coke deposits. Thereafter, the flow of air was discontinued and pure steam was passed through the reactor for 5 minutes before beginning the next experimental run.

For analyzing reaction products, the reactor effluent was cooled to ambient temperature (about 25°C (77°F)), and the uncondensed gaseous effluent was analyzed by gas chromatography. The main component of the uncondensed effluent was isobutene. Reactor conditions and test results demonstrated high isobutane conversion and high selectivity to isobutene, and also high butene-1 conversion and high selectivity to n-butane as shown below:

| | Run 1 | Run 2 |
|---|---|---|
| Steam flow (grams/hr.) | 1200 | -- |
| Hydrocarbon flow (cc/hr) | 3080 | 400 |
| LHSV | 4.1 | 0.54 |
| Stm./HC ratio | 2.1 | -- |
| Temperature °C (Deg.°F) | 593°C (1100) | 454°C (850) |
| Pressure Pa (psig) | 2.7 x 10⁶ (250) | 6.2 x 10⁵ (75) |
| Isobutane Conversion (%) | 50.4 | 18.2 |
| Selectivity to Isobutene (%)⁽¹⁾ | 91.1 | 88.5 |
| Run time (minutes) | 18 | 124 |
| Butene-1 conversion (%) | 82.3 | 91.6 |
| Selectivity to n-Butane (%)⁽²⁾ | 29.5 | 74.1 |

| | | |
|---|---|---|
| ⁽¹⁾ Yield of isobutene divided by conversion of isobutane X 100. | | |
| ⁽²⁾ Yield of n-butane divided by conversion of butene-1 X 100. | | |

### Example 2

The same experimental setup and procedure as in example 1, was used with a hydrocarbon feed stream composed as follows:

| | |
|---|---|
| isobutane | 57.77-% by weight, |
| n-butane | 2.00-% by weight, |
| n-butene-1 | 39.25-% by weight, |
| trans-2-butene | 0.05-% by weight, |
| cis-2-butene | 0.00-% by weight, |
| propane | 0.20-% by weight, |
| pentane | 0.03-% by weight, and |
| air | 0.70-% by weight |

Reactor conditions and obtained test results were as follows.

| | |
|---|---|
| Steam flow (grams/hr.) | -- |
| Hydrocarbon flow (cc/hr) | 400 |
| LHSV (cc/cc catalyst/hr) | 0.53 |
| Steam/HC ratio | -- |
| Temperature °C (Deg. °F) | 468 (875) |
| Pressure Pa (psig) | 6.2 x 10⁵ (75) |
| Isobutane conv. (%) | 11.2 |
| Selectivity to Isobutene (%) | 81.1 |
| Run time (minutes) | 57 |
| Butene-1 conversion (%) | 93.6 |
| Selectivity to n-Butane (%) | 75.6 |

The above test results show that the Pt, Sn on ZnAl₂O₄/CaAl₂O₄ is effective for simultaneously carrying out the required hydrogenation and dehydrogenation reactions in a single reaction zone. The hydrogen from the isobutane dehydrogenation was mostly consumed, as desired, by the hydrogenation of the linear butenes. This approach is highly advantageous because the high endothermicity of the dehydrogenation reaction can be balanced against the nearly equal exothermicity of the hydrogenation reaction, with most of the heat requirements satisfied through internal generation.

## Claims

1. A process for simultaneous hydrogenation of an olefin and dehydrogenation of a paraffin in a combination hydrogenation/dehydrogenation unit having a single reaction zone, said process comprising the steps of:
passing a structurally mixed hydrocarbon feed stream containing an isoparaffin having four or five carbon atoms per molecule and an n-olefin having a number of carbon atoms per molecule equal to said isoparaffin; and
contacting said structurally mixed hydrocarbon feed stream with a supported Group VIII noble metal catalyst in said combination reaction zone under conditions sufficient for conversion of a portion of said isoparaffin to an isoolefin and a portion of said n-olefin to an n-paraffin.

2. An integrated process for producing tertiary alkyl ether compounds which joins isoolefin production and ether production by reacting the produced isoolefin with a primary alcohol under etherification conditions, said integrated process comprising the steps of:
(a) passing a structurally mixed hydrocarbon feed stream containing an isoparaffin having four or five carbon atoms per molecule and an n-olefin, having a number of carbon atoms per molecule equal to said isoparaffin, to a combination hydrogenation/dehydrogenation unit;
(b) contacting said mixed hydrocarbon feed stream with a supported Group VIII noble metal catalyst in said combination unit under conditions sufficient for simultaneous conversion of a portion of said isoparaffin to an isoolefin and a portion of said n-olefin to an n-paraffin;
(c) withdrawing reaction product in a stream from said combination unit and passing at least a portion of said reaction product stream to an ether forming unit;
(d) providing a primary alcohol containing one or two carbon atoms per molecule to said ether forming unit via an alcohol feed stream; and
(e) reacting said isoolefin and said primary alcohol in said ether forming unit to form tertiary alkyl ether, and withdrawing tertiary alkyl ether from said ether forming unit in an ether product stream,
in particular wherein said isoparaffin is isobutane and said n-olefin is n-butene and said ether unit is an MTBE unit and said tertiary alkyl ether is MTBE.

3. A process in accordance with claim 2, wherein step (c) comprises passing said product stream to a distillation column;
(a) withdrawing an overhead stream from said distillation column and passing said overhead stream to an ether forming unit.

4. A process of claim 2 or 3 characterized by one or more of the following features:
(i) said primary alcohol is selected from a group of alcohols comprising methanol and ethanol.
(ii) said catalyst comprises zinc aluminate, platinum, and tin, in particular wherein said catalyst additionally comprises calcium aluminate.
(iii) said catalyst comprises about 0.5 weight % platinum, and about 0.1-5 weight % tin which is present as tin oxide.
(iv) said combination hydrogenation/dehydrogenation conditions in step (b) comprise a temperature in the range of from 426.6 to 648.8°C (800°F to 1200°F.)

5. A process in accordance with claim 2 additionally comprising providing a plurality of recycle streams from said ether forming unit to said combination unit, said plurality of recycle stream comprising a first recycle stream containing butene-2, a second recycle stream containing isobutane, and a third recycle stream containing isobutane, isobutene, and butene-1, in particular wherein said step of providing a plurality of recycle streams additionally comprises:
withdrawing a residual stream of remaining unreacted constituents comprising isobutane, isobutene, n-butane, n-butene-1 and n-butene-2 from said ether forming unit and passing said residual stream to a distillation tower for providing a bottom stream and an overhead stream;
passing said bottom stream containing n-butane and butene-2 to a separator;
withdrawing said first recycle stream from said separator and passing said first recycle stream from said separator to said combination unit;
withdrawing an n-butane stream from said separator and passing said n-butane stream to an isomerization unit for converting n-butane to isobutane;
withdrawing said second recycle stream from said isomerization unit and passing said second recycle stream to said combination unit; and
withdrawing said third recycle stream overhead from said distillation tower and passing said third recycle stream to said combination unit.

6. A process in accordance with claim 3 additionally comprising providing a plurality of recycle streams to said combination unit, said plurality of recycle stream comprising a first recycle stream containing butene-2, a second recycle stream containing isobutane, and a third recycle stream containing isobutane, isobutene, and butene-1, in particular wherein said catalyst comprises zinc aluminate, calcium aluminate, platinum, and tin, more particularly wherein said step of providing a plurality of recycle streams additionally comprises:
passing a bottom stream from the distillation column containing n-butane and butene-2 to a separator;
withdrawing said first recycle stream from said separator and passing said first recycle stream from said separator to said combination unit;
withdrawing an n-butane stream from said separator and passing said n-butane stream to an isomerization reactor for converting n-butane to isobutane;
withdrawing said second recycle stream from said isomerization unit and passing said second recyle stream to said combination unit; and
withdrawing said third recycle stream from said ether forming unit and passing said third recycle stream to said combination unit.

7. A process in accordance with claim 2 wherein said structurally mixed feed stream to said combination unit, containing isobutene, isobutane, and butene-1, is formed by separating n-butane and butene-2 from a source stream in a distillation tower and withdrawing said structurally mixed feed stream in an overhead stream from said distillation tower, said process additionally comprising:
contacting a bottom stream from said distillation tower containing n-butane and butene-2 with an acid catalyst under alkylation conditions in an alkylation unit to produce reaction constituents essentially comprising alkylate product and n-butane;
withdrawing an alkylate product and an n-butane stream from said alkylation unit;
passing said n-butane stream to an isomerization unit wherein n-butane is isomerized to isobutane; and
withdrawing a recycle stream comprising isobutane from said isomerization unit and passing said recyle stream to said combination reactor.

8. A process in accordance with claim 2 additionally comprising:
withdrawing a residual stream of remaining unreacted constituents from said MTBE unit and passing said residual stream containing isobutane, n-butene, and n-butane to an alkylation unit;
contacting said residual stream with an acid catalyst under alkylation conditions in an alkylation unit to produce reaction constituents essentially comprising alkylate product and n-butane;
withdrawing an alkylate product stream and an n-butane stream from said alkylation unit;
passing said n-butane stream to an isomerization unit wherein n-butane is isomerized to isobutane; and
withdrawing a recycle stream comprising isobutane from said isomerization unit and passing said recycle stream to said combination reactor.

9. A process in accordance with claim 2 wherein said structurally mixed hydrocarbon feedstream to said combination unit is formed from a first portion of a residual stream of remaining unreacted constituents withdrawn from said MTBE unit, said process additionally comprising the following steps:
passing a second portion of said residual stream from said MTBE unit to an alkylation unit;
contacting said residual stream with an acid catalyst under alkylation conditions in an alkylation zone to produce reaction constituents essentially comprising alkylate product and n-butane;
withdrawing an alkylate product stream and an n-butane stream from said alkylation zone;
passing said n-butane stream to an isomerization unit wherein n-butane is isomerized to isobutane; and
withdrawing a recycle stream comprising isobutane from said isomerization unit and passing said recycle stream to said combination reactor.

10. A process in accordance with claim 2 additionally comprising the following steps:
withdrawing a residual stream of remaining unreacted constituents from said MTBE unit;
passing a first portion of said residual stream to an alkylation unit and passing a second portion of said residual stream to a separation unit;
separating n-butene from n-butane and isobutane in said separation unit;
withdrawing n-butene from said separation unit and passing n-butene in a first recycle stream to said combination unit;
contacting said first portion of said residual stream with an acid catalyst under alkylation conditions in an alkylation unit to produce reaction constituents essentially comprising alkylate product and n-butane;
withdrawing an alkylate product stream and an n-butane stream from said alkylation unit;
passing said n-butane stream to an isomerization unit wherein n-butane is isomerized to isobutane; and
withdrawing a second recycle stream comprising isobutane from said isomerization unit and passing said second recycle stream to said combination reactor.

11. A process in accordance with claim 2, 3 or 4 wherein said isoparaffin is isopentane, said n-olefin is n-pentene, said ether forming unit is an MTAE unit and said tertiary alkyl ether is MTAE.

## Patentansprüche

1. Verfahren zur gleichzeitigen Hydrierung eines Olefins und Dehydrierung eines Paraffins in einer Kombinationshydrier/Dehydriereinheit mit einer einzigen Reaktionszone, wobei das Verfahren folgende Stufen umfaßt:
Leiten eines strukturell gemischten Kohlenwasserstoffeinsatzmaterialstroms, der ein Isoparaffin mit vier oder fünf Kohlenstoffatomen pro Molekül und ein n-Olefin mit einer Anzahl von Kohlenstoffatomen pro Molekül, die gleich der des Isoparaffins ist, enthält, und
Kontaktieren des strukturell gemischten Kohlenwasserstoffeinsatzmaterialstroms mit einem trägergebundenen Gruppe VIII-Edelmetallkatalysator in der Kombinationsreaktionszone unter Bedingungen, die geeignet für die Umwandlung eines Anteils des Isoparaffins in ein Isoolefin und eines Anteils des n-Olefins in ein n-Paraffin sind.

2. Integriertes Verfahren für die Herstellung tertiärer Alkyletherverbindungen, das die Isoolefinherstellung und die Etherherstellung durch Umsetzung des gebildeten Isoolefins mit einem primären Alkohol unter Veretherungsbedingungen verknüpft, wobei das integrierte Verfahren folgende Stufen umfaßt:
(a) Leiten eines strukturell gemischten Kohlenwasserstoffeinsatzmaterialstroms, der ein Isoparaffin mit vier oder fünf Kohlenstoffatomen pro Molekül und ein n-Olefin mit einer Anzahl von Kohlenstoffatomen pro Molekül, die gleich der des Isoparaffins ist, enthält, in eine Kombinationshydrier/Dehydriereinheit;
(b) Kontaktieren des gemischten Kohlenwasserstoffeinsatzmaterialstroms mit einem trägergebundenen Gruppe VIII-Edelmetallkatalysator in der Kombinationseinheit unter Bedingungen, die für die gleichzeitige Umwandlung eines Anteils des Isoparaffins in ein Isoolefin und eines Anteils des n-Olefins in ein n-Paraffin geeignet sind;
(c) Abziehen von Reaktionsprodukt in einem Strom aus der Kombinationseinheit und Leiten mindestens eines Anteils des Reaktionsproduktstroms in eine Etherbildungseinheit;
(d) Bereitstellung eines primären Alkohols, der ein oder zwei Kohlenstoffatome pro Molekül enthält, in der Etherbildungseinheit über einen Alkoholeinsatzmaterialstrom; und
(e) Umsetzung des Isoolefins und des primären Alkohole in der Etherbildungseinheit unter Bildung eines tertiären Alkylethers und Abziehen des tertiären Alkylethers aus der Etherbildungseinheit in einem Etherproduktstrom,
insbesondere wobei es sich bei dem Isoparaffin um Isobutan und bei dem n-olefin um n-Buten handelt und die Ethereinheit eine MTBE-Einheit ist und es sich bei dem tertiären Alkylether um MTBE handelt.

3. Verfahren nach Anspruch 2, wobei Stufe (c) das Leiten des Produktstroms in eine Destillationskolonne;
(a) das Abziehen eines Kopfproduktstroms aus der Destillationskolonne und das Leiten des Kopfproduktstroms in eine Etherbildungseinheit umfaßt.

4. Verfahren nach Anspruch 2 oder 3, gekennzeichnet durch eines oder mehrere der folgenden Merkmale:
(i) der primäre Alkohol ist aus einer Gruppe von Alkoholen ausgewählt, die Methanol und Ethanol umfaßt;
(ii) der Katalysator umfaßt Zinkaluminat, Platin und Zinn, wobei der Katalysator insbesondere zusätzlich Calciumaluminat umfaßt;
(iii) der Katalysator umfaßt etwa 0,5 Gew.-% Platin und etwa 0,1 bis 5 Gew.-% Zinn, das als Zinnoxid vorliegt;
(iv) die Kombinationshydrier/Dehydrierbedingungen in Stufe (b) umfassen eine Temperatur im Bereich von 426,6°C bis 648,8°C (800°F bis 1200°F).

5. Verfahren nach Anspruch 2, das zusätzlich die Bereitstellung einer Mehrzahl von Rückführströmen aus der Etherbildungseinheit in die Kombinationseinheit umfaßt, wobei die Mehrzahl der Rückführströme einen ersten Rückführstrom, der Buten-2 enthält, einen zweiten Rückführstrom, der Isobutan enthält, und einen dritten Rückführstrom, der Isobutan, Isobuten und Buten-1 enthält, umfaßt, insbesondere wobei die Stufe der Bereitstellung einer Mehrzahl von Rückführströmen zusätzlich folgendes umfaßt:
Abziehen eines Reststroms von restlichen, nicht-umgesetzten Bestandteilen, der Isobutan, Isobuten, n-Butan, n-Buten-1 und n-Buten-2 umfaßt, aus der Etherbildungseinheit und Leiten des Reststroms in einen Destillationsturm zur Bereitstellung eines Sumpfstroms und eines Kopfproduktstroms;
Leiten des Sumpfstroms, der n-Butan und Buten-2 enthält, in eine Trenneinrichtung;
Abziehen des ersten Rückführstroms aus der Trenneinrichtung und Leiten des ersten Rückführstroms von der Trenneinrichtung in die Kombinationseinheit;
Abziehen eines n-Butanstroms aus der Trenneinrichtung und Leiten des n-Butanstroms in eine Isomerisierungseinheit zur Umwandlung von n-Butan in Isobutan;
Abziehen des zweiten Rückführstroms aus der Isomerisierungseinheit und Leiten des zweiten Rückführstroms in die Kombinationseinheit; und
Abziehen des dritten Rückführstrom-Kopfprodukts aus dem Destillationsturm und Leiten des dritten Rückführstroms in die Kombinationseinheit.

6. Verfahren nach Anspruch 3, das zusätzlich die Bereitstellung einer Mehrzahl von Rückführströmen zur Kombinationseinheit umfaßt, wobei die Mehrzahl der Rückführströme einen ersten Rückführstrom, der Buten-2 enthält, einen zweiten Rückführstrom, der Isobutan enthält, und einen dritten Rückführstrom, der Isobutan, Isobuten und Buten-1 enthält, umfaßt, speziell wobei der Katalysator Zinkaluminat, Calciumaluminat, Platin und Zinn umfaßt und insbesondere wobei die Stufe der Bereitstellung einer Mehrzahl von Rückführströmen zusätzlich folgendes umfaßt:
Leiten eines Sumpfstroms aus der Destillationskolonne, der n-Butan und Buten-2 enthält, in eine Trenneinrichtung;
Abziehen des ersten Rückführstroms aus der Trenneinrichtung und Leiten des ersten Rückführstroms aus der Trenneinrichtung in die Kombinationseinheit;
Abziehen eines n-Butanstroms aus der Trenneinrichtung und Leiten des n-Butanstroms in einen Isomerisierungsreaktor für die Umwandlung von n-Butan in Isobutan;
Abziehen des zweiten Rückführstroms aus der Isomerisierungseinheit und Leiten des zweiten Rückführstroms in die Kombinationseinheit; und
Abziehen des dritten Rückführstroms aus der Etherbildungseinheit und Leiten des dritten Rückführstroms zur Kombinationseinheit.

7. Verfahren nach Anspruch 2, wobei der strukturell gemischte Einsatzmaterialstrom zur Kombinationseinheit, der Isobuten, Isobutan und Buten-1 enthält, durch Abtrennung von n-Butan und Buten-2 aus einem Quellstrom in einem Destillationsturm und Abziehen des strukturell gemischten Einsatzmaterialstroms in einem Kopfproduktstrom aus dem Destillationsturm gebildet wird, wobei das Verfahren zusätzlich folgendes umfaßt:
Kontaktieren eines Sumpfstroms aus dem Destillationsturm, der n-Butan und Buten-2 enthält, mit einem Säurekatalysator unter Alkylierungsbedingungen in einer Alkylierungseinheit unter Bildung von Reaktionsbestandteilen, die im wesentlichen Alkylatprodukt und n-Butan umfassen;
Abziehen eines Alkylatprodukts und eines n-Butanstroms aus der Alkylierungseinheit;
Leiten des n-Butanstroms in eine Isomerisierungseinheit, wo n-Butan zu Isobutan isomerisiert wird; und
Abziehen eines Rückführstroms, der Isobutan umfaßt, aus der Isomerisierungseinheit und Leiten des Rückführstroms in den Kombinationsreaktor.

8. Verfahren nach Anspruch 2, das zusätzlich folgendes umfaßt:
Abziehen eines Reststroms von restlichen, nicht-umgesetzten Bestandteile aus der MTBE-Einheit und Leiten des Reststroms, der Isobutan, n-Buten und n-Butan enthält, in eine Alkylierungseinheit;
Kontaktieren des Reststroms mit einem Säurekatalysator unter Alkylierungsbedingungen in einer Alkylierungseinheit unter Bildung von Reaktionsbestandteilen, die im wesentlichen Alkylatprodukt und n-Butan umfassen;
Abziehen eines Alkylatproduktstroms und eines n-Butanstroms aus der Alkylierungseinheit;
Leiten des n-Butanstroms in eine Isomerisierungseinheit, wo n-Butan zu Isobutan isomerisiert wird; und
Abziehen eines Rückführstroms, der Isobutan umfaßt, aus der Isomerisierungseinheit und Leiten des Rückführstroms in den Kombinationsreaktor.

9. Verfahren nach Anspruch 2, wobei der strukturell gemischte Kohlenwasserstoffeinsatzmaterialstrom zu der Kombinationseinheit aus einem ersten Anteil eines Reststroms von restlichen, nicht-umgesetzten Bestandteilen, die aus der MTBE-Einheit abgezogen werden, gebildet wird, wobei das Verfahren zusätzlich folgende Stufen umfaßt:
Leiten eines zweiten Anteils des Reststroms aus der MTBE-Einheit in eine Alkylierungseinheit;
Kontaktieren des Reststroms mit einem Säurekatalysator unter Alkylierungsbedingungen in einer Alkylierungszone unter Bildung von Reaktionsbestandteilen, die im wesentlichen Alkylatprodukt und n-Butan umfassen;
Abziehen eines Alkylatproduktstroms und eines n-Butanstroms aus der Alkylierungszone;
Leiten des n-Butanstroms in eine Isomerisierungseinheit, wo n-Butan zu Isobutan isomerisiert wird; und
Abziehen eines Rückführstroms, der Isobutan umfaßt, aus der Isomerisierungseinheit und Leiten des Rückführstroms in den Kombinationsreaktor.

10. Verfahren nach Anspruch 2, das zusätzlich folgende Stufen umfaßt:
Abziehen eines Reststroms von restlichen, nicht-umgesetzten Bestandteilen aus der MTBE-Einheit;
Leiten eines ersten Anteils des Reststroms in eine Alkylierungseinheit und Leiten eines zweiten Anteils des Reststroms in eine Trenneinheit;
Trennen von n-Buten von n-Butan und Isobutan in der Trenneinheit;
Abziehen von n-Buten aus der Trenneinheit und Leiten von n-Buten in einem ersten Rückführstrom in die Kombinationseinheit;
Kontaktieren des ersten Anteils des Reststroms mit einem Säurekatalysator unter Alkylierungsbedingungen in einer Alkylierungseinheit unter Bildung von Reaktionsbestandteilen, die im wesentlichen Alkylatprodukt und n-Butan umfassen;
Abziehen eines Alkylatproduktstroms und eines n-Butanstroms aus der Alkylierungseinheit;
Leiten des n-Butanstroms in eine Isomerisierungseinheit, wobei n-Butan zu Isobutan isomerisiert wird; und
Abziehen eines zweiten Rückführstroms, der Isobutan umfaßt, aus der Isomerisierungseinheit und Leiten des zweiten Rückführstroms in den Kombinationsreaktor.

11. Verfahren nach Anspruch 2, 3 oder 4, wobei es sich bei dem Isoparaffin um Isopentan handelt, es sich bei dem n-Olefin um n-Penten handelt, die Etherbildungseinheit eine MTAE-Einheit ist und es sich bei dem tertiären Alkylether um MTAE handelt.

## Revendications

1. Un procédé pour l'hydrogénation d'une oléfine et la déshydrogénation d'une paraffine simultanées dans une unité combinée d'hydrogénation/déshydrogénation ayant une seule zone de réaction, ledit procédé comprenant les étapes consistant à :
faire passer un courant de charge hydrocarbonée structurellement mélangée contenant une isoparaffine ayant quatre ou cinq atomes de carbone par molécule et une n-oléfine ayant un nombre d'atomes de carbone par molécule égal à ladite isoparaffine; et
mettre en contact ledit courant de charge hydrocarbonée structurellement mélangée avec un catalyseur de métal noble du Groupe VIII fixé sur un support dans ladite zone de réaction combinée sous des conditions suffisantes pour transformer une partie de ladite isoparaffine en une isooléfine et une partie de ladite n-oléfine en une n-paraffine.

2. Un procédé intégré pour la production de composés de tertio-alkyl-éther qui réunit la production d'isooléfine et la production d'éther en faisant réagir l'isooléfine produite avec un alcool primaire sous des conditions d'éthérification, ledit procédé intégré comprenant les étapes consistant à :
(a) faire passer un courant de charge hydrocarbonée structurellement mélangée contenant une isoparaffine ayant quatre ou cinq atomes de carbone par molécule et une n-oléfine ayant un nombre d'atomes de carbone par molécule égal à ladite isoparaffine dans une unité combinée d'hydrogénation/déshydrogénation;
(b) mettre en contact ledit courant de charge hydrocarbonée mélangée avec un catalyseur de métal noble du Groupe VIII fixé sur un support dans ladite unité combinée sous des conditions suffisantes pour transformer simultanément une partie de ladite isoparaffine en une isooléfine et une partie de ladite n-oléfine en une n-paraffine;
(c) retirer le produit de réaction dans un courant à partir de ladite unité combinée et faire passer au moins une partie dudit courant de produit de réaction dans une unité de formation d'éther;
(d) prévoir un alcool primaire contenant un ou deux atomes de carbone par molécule à ladite unité de formation d'éther par l'intermédiaire d'un courant de charge d'alcool; et
(e) faire réagir ladite isooléfine et ledit alcool primaire dans ladite unité de formation d'éther pour former un tertio-alkyl-éther et retirer le tertio-alkyl-éther de ladite unité de formation d'éther dans un courant de produit d'éther;
en particulier dans lequel ladite isoparaffine est l'isobutane et ladite n-oléfine est le n-butène et ladite unité d'éther est une unité MTBE et ledit tertio-alkyl-éther est le MTBE.

3. Un procédé selon la revendication 2, dans lequel l'étape (c) comprend le passage dudit courant de produit dans une colonne de distillation;
(a) le retrait d'un courant de produit aérien à partir de ladite colonne de distillation et le passage dudit courant de produit aérien à une unité de formation d'éther.

4. Un procédé selon la revendication 2 ou 3, caractérisé par une ou plusieurs des caractéristiques suivantes:
(i) ledit alcool primaire est choisi parmi un groupe d'alcools comprenant le méthanol et l'éthanol;
(ii) ledit catalyseur comprend de l'aluminate de zinc, du platine et de l'étain, en particulier dans lequel ledit catalyseur comprend supplémentairement de l'aluminate de calcium;
(iii) ledit catalyseur comprend environ 0,5% en poids de platine et environ 0,1 à 5% en poids d'étain qui est présent sous forme d'oxyde d'étain;
(iv) lesdites conditions combinées d'hydrogénation/déshydrogénation dans l'étape (b) comprennent une température comprise dans la gamme de 426,6 à 648,8°C (800°F à 1200°F).

5. Un procédé selon la revendication 2, comprenant supplémentairement la prévision d'une pluralité de courants de recyclage à partir de ladite unité de formation d'éther à ladite unité combinée, ladite pluralité de courants de recyclage comprenant un premier courant de recyclage contenant du butène-2, un deuxième courant de recyclage contenant de l'isobutane et un troisième courant de recyclage contenant de l'isobutane, de l'isobutène et du butène-1 en particulier dans lequel ladite étape de prévision d'une pluralité de courants de recyclage comprend supplémentairement:
le retrait d'un courant résiduaire des constituants restants n'ayant pas réagi comprenant de l'isobutane, de l'isobutène, du n-butane, du n-butène-1 et du n-butène 2 à partir de ladite unité de formation d'éther et le passage dudit courant résiduaire à une tour de distillation pour obtenir un courant de produit de queue et un courant de produit aérien;
le passage dudit courant de produit de queue contenant du n-butane et du butène-2 à un séparateur;
le retrait dudit premier courant de recyclage à partir dudit séparateur et le passage dudit premier courant de recyclage à partir dudit séparateur à ladite unité combinée;
le retrait d'un courant de n-butane à partir dudit séparateur et le passage dudit courant de n-butane à une unité d'isomérisation pour la transformation de n-butane en isobutane;
le retrait dudit deuxième courant de recyclage à partir de ladite unité d'isomérisation et le passage dudit deuxième courant de recyclage à ladite unité combinée; et
le retrait dudit troisième courant de recyclage de produit aérien à partir de ladite tour de distillation et le passage dudit troisième courant de recyclage à ladite unité combinée.

6. Un procédé selon la revendication 3, comprenant supplémentairement la prévision d'une pluralité de courants de recyclage à ladite unité combinée, ladite pluralité de courants de recyclage comprenant un premier courant de recyclage contenant du butène-2, un deuxième courant de recyclage contenant de l'isobutane et un troisième courant de recyclage contenant de l'isobutane, de l'isobutène et du butène-1, en particulier dans lequel ledit catalyseur comprend de l'aluminate de zinc, de l'aluminate de calcium, du platine et de l'étain, plus particulièrement dans lequel ladite étape de prévision d'une pluralité de courants de recyclage comprend supplémentairement :
le passage d'un courant de produit de queue à partir de la colonne de distillation contenant le n-butane et le butène-2 à un séparateur;
le retrait dudit premier courant de recyclage à partir dudit séparateur et le passage dudit premier courant de recyclage à partir dudit séparateur à ladite unité combinée;
le retrait d'un courant de n-butane à partir dudit séparateur et le passage dudit courant de n-butane à un réacteur d'isomérisation pour transformer le n-butane en isobutane;
le retrait dudit deuxième courant de recyclage à partir de ladite unité d'isomérisation et le passage dudit deuxième courant de recyclage à ladite unité combinée; et
le retrait dudit troisième courant de recyclage à partir de ladite unité de formation d'éther et le passage dudit troisième courant de recyclage à ladite unité combinée.

7. Un procédé selon la revendication 2, dans lequel ledit courant de charge mélangée structurellement à ladite unité combinée contenant de l'isobutène, de l'isobutane et du butène-1 est formé en séparant du n-butane et du butène-2 à partir d'un courant d'alimentation dans une tour de distillation et en retirant ledit courant de charge mélangée structurellement selon un courant de produit aérien à partir de ladite tour de distillation, ledit procédé comprenant supplémentairement:
la mise en contact d'un courant de produit de queue à partir de ladite tour de distillation contenant du n-butane et du butène-2 avec un catalyseur acide sous des conditions d'alkylation dans une unité d'alkylation pour produire des constituants de réaction comprenant essentiellement un alkylat et du n-butane;
le retrait d'un alkylat et d'un courant de n-butane à partir de ladite unité d'alkylation;
le passage dudit courant de n-butane à une unité d'isomérisation dans laquelle le n-butane est isomérisé en isobutane; et
le retrait d'un courant de recyclage comprenant de l'isobutane à partir de ladite unité d'isomérisation et le passage dudit courant de recyclage audit réacteur combiné.

8. Un procédé selon la revendication 2 comprenant supplémentairement:
le retrait d'un courant résiduaire de constituants n'ayant pas réagi restants à partir de ladite unité MTBE et le passage dudit courant résiduaire contenant de l'isobutane, du n-butène et du n-butane à une unité d'alkylation;
la mise en contact dudit courant résiduaire avec un catalyseur acide sous des conditions d'alkylation dans une unité d'alkylation pour produire des constituants de réaction comprenant essentiellement un alkylat et du n-butane;
le retrait d'un courant d'alkylat et d'un courant de n-butane à partir de ladite unité d'alkylation;
le passage dudit courant de n-butane à une unité d'isomérisation où le n-butane est isomérisé en isobutane; et
le retrait d'un courant de recyclage comprenant de l'isobutane à partir de ladite unité d'isomérisation et le passage dudit courant de recyclage audit réacteur combiné.

9. Un procédé selon la revendication 2, dans lequel ledit courant de charge hydrocarbonée mélangée structurellement à ladite unité combinée est formé à partir d'une première partie d'un courant résiduaire de constituants n'ayant pas réagi restants retirés à partir de ladite unité MTBE, ledit procédé comprenant supplémentairement les étapes suivantes:
le passage d'une seconde partie dudit courant résiduaire à partir de ladite unité MTBE à une unité d'alkylation;
la mise en contact dudit courant résiduaire avec un catalyseur acide sous des conditions d'alkylation dans une zone d'alkylation pour produire des constituants de réaction comprenant essentiellement l'alkylat et du n-butane;
le retrait d'un courant d'alkylat et d'un courant de n-butane à partir de ladite zone d'alkylation;
le passage dudit courant de n-butane à une unité d'isomérisation où le n-butane est isomérisé en isobutane; et
le retrait d'un courant de recyclage comprenant de l'isobutane à partir de ladite unité d'isomérisation et le passage dudit courant de recyclage audit réacteur combiné.

10. Un procédé selon la revendication 2 comprenant supplémentairement les étapes suivantes:
le retrait d'un courant résiduaire de constituants n'ayant pas réagi restants à partir de ladite unité MTBE;
le passage d'une première partie dudit courant résiduaire à une unité d'alkylation et le passage d'une deuxième partie dudit courant résiduaire à une unité de séparation;
la séparation du n-butène à partir du n-butane et de l'isobutane dans ladite unité de séparation;
le retrait du n-butène à partir de ladite unité de séparation et le passage du n-butène dans un premier courant de recyclage à ladite unité combinée;
la mise en contact de ladite première partie dudit courant résiduaire avec un catalyseur acide sous des conditions d'alkylation dans une unité d'alkylation pour produire des constituants de réaction comprenant essentiellement un alkylat et du n-butane;
le retrait d'un courant d'alkylat et d'un courant de n-butane à partir de ladite unité d'alkylation;
le passage dudit courant de n-butane à une unité d'isomérisation dans laquelle le n-butane est isomérisé en isobutane; et
le retrait d'un second courant de recyclage comprenant de l'isobutane à partir de ladite unité d'isomérisation et le passage dudit deuxième courant de recyclage audit réacteur combiné.

11. Un procédé selon la revendication 2, 3 ou 4, dans lequel ladite isoparaffine est l'isopentane, ladite n-oléfine est le n-pentène et ladite unité de formation d'éther est une unité MTAE et ledit tertio-alkyl éther est le MTAE.
